# EUROPEAN PATENT SPECIFICATION

(11) **EP 4 353 281 B1**
(45) Date of publication and mention of the grant of the patent: **06.08.2025**
(21) Application number: 24159836.6
(22) Date of filing: 25.09.2019
(51) Int. Cl.: A61M 5/32, A61M 5/178

(54) **SYRINGE WITH FLAT INDICIA DISPLAY SURFACE**
SPRITZE MIT FLACHER MARKIERUNGSANZEIGEOBERFLÄCHE
SERINGUE AYANT UNE SURFACE D'AFFICHAGE D'INDICES PLATE

(30) Priority: 29.03.2019 US 201916370239
(43) Date of publication of application: 17.04.2024
(62) Divisional of application: 19923319.8
(73) Proprietor: Retractable Technologies, Inc., Little Elm, TX 75068 (US); Shaw, Thomas J., Frisco, TX 75034 (US)
(72) Inventor: SMALL, Mark, Heavener, 74937 (US); SHAW, Thomas J., Frisco, 75034 (US)
(74) Representative: Grünecker Patent- und Rechtsanwälte PartG mbB

(56) References cited:
- CN-A- 108 619 594
- US-A1- 2003 028 171
- US-B1- 6 213 987

## Description

### BACKGROUND OF THE INVENTION

### 1. Field of the Invention

This invention relates to a syringe configured for medical use in aspirating or injecting fluids. The syringe desirably includes a barrel, a plunger slidably engaging a portion of the barrel, a forwardly projecting needle. The barrel desirably has a substantially tubular inside surface, which functions as a side wall of a fluid chamber, and a longitudinally extending, substantially flat indicia display surface disposed on at least one side of the barrel. Preferably, the subject syringe includes two opposed, outwardly facing, longitudinally extending, substantially flat indicial display surfaces suitable for use in printing dosage scales on the syringe. Further relevant prior art is described in US 2003/028171 A1, US 6 213 987 B1 and CN 108 619 594 A.

According to the present invention, it is provided a syringe having the features defined in claim 1. Further preferred embodiments are defined in the dependent claims. In the following, there are described some arrangements, however, the scope of protection is defined by the claims. Some of the arrangements described in the following are helpful for understanding the present invention. One arrangement also includes a needle retraction mechanism and a laterally offset needle retraction cavity that is substantially coextensive with the length of the fluid chamber of the barrel. The needle is selectively retracted following an injection by sliding the barrel and needle retraction cavity transversely relative to the needle, thereby moving the needle retraction cavity laterally into coaxial alignment with the needle. The substantially full-length, laterally offset needle retraction cavity facilitates the optional use of longer retractable needles than are usable with conventional safety syringes having retractable needles and broadens the range of uses and procedures that are performable with the device without increasing the overall length of the syringe. Depending upon needle length, such uses and procedures can include, for example, performing spinal taps, administering epidural anesthesia, aspirating cysts, and the like, as well as for administering intradermal, subcutaneous or intramuscular injections.

Another aspect relates to a syringe having a barrel comprising a substantially cylindrical fluid chamber, a needle retraction cavity disposed in parallel and laterally spaced-apart relation to the fluid chamber, and at least one substantially flat, outwardly facing display surface to which indicia such as dosage scales can be applied using a conventional pad printing process. Still another aspect relates to a syringe having a barrel with two oppositely facing, substantially flat surfaces on which the same or different indicia can be pad printed without having to rotate the barrel, even when printing on 1 mL, 0.5 mL or smaller syringes. The substantially flat display surfaces also facilitate the application of indicia to a syringe by other processes such as embossing, injection molding, and the like.

Another aspect relates to a medical syringe comprising a barrel and plunger as disclosed above in combination with a frontal attachment having a forwardly projecting, rearwardly biased needle. The frontal attachment and barrel are desirably cooperatively configured so that the frontal attachment slidably engages a front portion of the barrel along an axis that is transverse to the longitudinal axis through the needle.

Another arrangement as described in relation to paragraph **[0001]** above embodies a needle safety device having a needle tip shield that extends circumferentially around and is desirably coaxially aligned with the needle. The needle tip shield is desirably connected to or unitarily molded together with an elongate activation handle that is also part of the needle safety device and slidably engages the barrel. The needle safety device of this arrangement eliminates the need for having a needle retraction mechanism and a needle retraction cavity, and does not require transverse sliding movement of the barrel relative to the needle to protect users from accidental needle sticks. Following use of this arrangement of the syringe, the needle tip shield is selectively advanced to protect the user from the forwardly projecting needle tip by applying manual pressure to a touch surface of the activation handle that is located rearwardly of the needle and needle tip shield. The activation handle slidably engages the syringe barrel and is forwardly slidable relative to the barrel from a first position, in which the needle tip is uncovered, to a second position in which the needle tip is surrounded and protected against inadvertent needle sticks by the needle tip shield. Because the forwardly slidable needle safety device of this arrangement embodies a stop surface that prevents subsequent rearward movement of the device relative to the barrel of the syringe, the user is protected against subsequent accidental exposure of the needle tip and associated needle stick injuries. This arrangement combines the advantages of flat, printable surfaces having more easily readable dosage indicia with the cost advantages of a simpler but still effective and easy-to-use needle safety device to provide a safe and more affordable solution, particularly for persons needing frequent injections to treat various chronic health conditions. Furthermore, because the dosage markings and indicia are more easily readable by the user, there is less risk of administering an incorrect dosage of a medicinal fluid to a patient and thereby less risk of causing other unintended consequences.

### 2. Description of Related Art

Syringes intended for medical use typically have barrels comprising substantially cylindrical inside and outside walls, meaning that volumetric dosage indicia or other markings are applied to an arcuate outer surface during manufacture. This can be difficult, and especially so when the diameter of the barrel and radius of curvature of the outside wall are small (as with 1 mL, 0.5 mL and smaller syringes) and where the available outside surface area is extremely limited, or on syringes where different dosing scales or other indicia are applied to opposite sides of the barrel. In such instances it is often necessary to spin or rotate the barrel while printing, and it is also often difficult to read indicia such as dose measuring lines and the related numeric values or other markings because they wrap so far around the circumference of the barrel and because indicia or markings on one side are often viewable through the syringe and can confuse the user, sometimes leading to the commission of medical errors. For at least these reasons, syringes having substantially flat surfaces for use in applying dosage markings and other indicia are needed.

Medical syringes having rearwardly biased needles that retract into coaxially aligned retraction cavities disposed inside the plunger are known, having previously been disclosed, for example, in United States Patent Nos. 5,049,133; 5,053,010; 5,084,018 and 6,090,077. More recently, medical syringes having frontal attachments containing rearwardly biased needles that retract into needle retraction cavities that are part of the frontal attachment have been disclosed, for example, in U.S. 9,381,309.

Even more recently, medical syringes with frontal attachments have been disclosed that slidably engage barrels having needle retraction cavities unitarily molded together with the barrel and disposed parallel to the fluid chamber. In U.S. 9,814,841 (FIGS. 37-42), for example, the length of the needle retraction cavity is substantially shorter than the length of the fluid chamber inside the barrel and the needle retraction cavity does not cooperate with an outer wall of the barrel to form substantially flat outside surfaces that are adjacent to and substantially coextensive with the fluid chamber.

Other references disclosing devices arguably bearing some degree of similarity to various aspects or arrangements include the following: U.S. 4,573,976; U.S. 4,702,738; U.S. 4,790,828; U.S. 4,915,696; U.S. 5,037,402; U.S. 5,092,461; U.S. 5,215,534; U.S. 5,312,372; U.S. 9,623,192; U.S. Pub. No. 2002/0065488 A1; U.S. Pub. No. 2003/0038171 A1; and U.S. Pub. No. 2005/0159706 A1

### SUMMARY OF THE INVENTION

In particular, it is provided a syringe having the features defined in claim 1. Further preferred embodiments are defined in the dependent claims. According to one embodiment of the disclosure a syringe for medical use is disclosed that comprises a one-piece barrel having a fluid chamber and a laterally offset needle retraction cavity that are substantially parallel and separated by a common wall. The needle retraction cavity can have a non-circular cross-section and is desirably substantially coextensive in length with the fluid chamber, thereby facilitating the use of longer needles without increasing the overall length of the barrel in order to accommodate retraction of the longer needles as is the case with safety syringes that retract the needle into the barrel following use. The barrel further comprises an outer wall having at least one substantially flat, outwardly facing display surface upon which indicia such as a volumetric dosage scale, for example, can be applied using conventional pad printing technology or another similarly effective alternative means. The at least one substantially flat display surface is desirably disposed proximally to the fluid chamber and also desirably comprises a measurement scale that is longitudinally aligned with at least that portion of the fluid chamber that is useful for injecting or aspirating a fluid. According to one preferred arrangement two oppositely facing, longitudinally coextensive, substantially flat surfaces are provided, with each surface spanning at least a portion of an external wall of the fluid chamber and at least a portion of the external wall of the needle retraction cavity.

The subject syringe is particularly useful with syringes having usable volumes of 1 mL or less, which syringes typically have smaller barrel diameters that cause the volumetric dosage indicia applied to the outside surface to wrap around a greater portion of the circumference of the barrel. According to another preferred arrangement, pad printing technology (sometimes referred to as "tampography") is used to apply volumetric dosage indicia or other markings to the substantially flat display surface of the syringe. The needle retraction cavity can be made (preferably molded from a suitable polymeric material) with a non-circular cross-section and is offset laterally from the barrel to facilitate creation of a wider, outwardly facing, substantially flat display surface during manufacture. The substantially flat display surface desirably comprises at least one side that is proximal to the fluid chamber of the barrel and thereby provides an opportunity, if desired, for molding volumetric dosage markings or other indicia onto the otherwise flat display surface or embossing the indicia on or into the display surface.

Another arrangement of the subject syringe comprises a barrel and plunger as disclosed above in combination with a frontal attachment having a forwardly projecting, rearwardly biased needle. The frontal attachment and barrel are desirably cooperatively configured so that the frontal attachment slidably engages a front portion of the barrel along an axis that is transverse to the longitudinal axis through the needle. When the syringe is disposed in the use position, the needle is aligned with a first opening in the front of the barrel that is communicates with a substantially cylindrical fluid chamber inside the barrel to establish a coaxially aligned fluid flow path between the fluid chamber and the needle. A fluid seal is desirably seated around the first opening to resist fluid leakage between the frontal attachment and the barrel. The needle retraction cavity extends rearwardly from a second opening in the front of the barrel.in parallel and spaced-apart relation to the fluid chamber in the barrel, and desirably shares a common wall with at least a portion of fluid chamber. Following use of the syringe, relative transverse movement between the barrel and the frontal attachment repositions the rearwardly biased needle into alignment with the needle retraction cavity, thereby releasing the needle to be forced by the biasing means, typically a compressed spring, into a retracted position wherein the needle no longer projects forwardly from the syringe.

Another arrangement of the subject syringe embodies wider, substantially flat display surfaces disposed on a medical syringe having a unitary barrel and needle retraction cavity that cooperate with substantially flat edge portions of the surrounding flange to help prevent the syringe from rolling off a tray or other flat surface. The substantially flat surfaces also allow the flange around the barrel to be proportionally narrower as compared to the flanges of conventional syringes having tubular barrels and still provide larger surface areas that are more easily graspable by a user. The oppositely facing, substantially flat surfaces also improve stability and the degree of control that can be exercised over the syringe by a user during an injection or other procedure. Because the subject syringe has a barrel with a laterally offset needle retraction cavity that can be molded integrally with the fluid chamber, at least one, and preferably two (oppositely facing), substantially flat, outwardly facing surface areas are provided that are useful for the placement of an array comprising a volumetric scale or other indicia that are easily readable by the user and thereby reduce the likelihood of dosing errors during aspiration or injection.

Another arrangement comprises the subject syringe in combination with a selectively releasable needle cover that can also comprise a locking member configured to engage a portion of the barrel and resist sliding lateral movement of the barrel relative to the frontal attachment to prevent accidental retraction of the needle prior to using the syringe. Retraction of the needle following use of the syringe reduces the likelihood of reusing the syringe or of accidental needle sticks and the inadvertent transmission of blood-borne pathogens.

Another arrangement comprises the subject syringe in combination with a plunger cap that is releasably attached to the rear portion of the syringe, typically behind the finger flange, and is selectively removable prior to fluid aspiration or use. When both the needle cover and the plunger cap are in place, the needle and the internal, fluid-contacting portions of the syringe are enclosed and protected from contamination whether or not the syringe is also enclosed inside another package. Because of this, the syringes can be assembled and shipped in bulk prior to packaging and sterilization.

Although the subject syringe is especially useful in administering relatively small doses of a medicinal fluid such as insulin or a vaccine to a user by injection or infusion, the structure and operation of the apparatus is not limited to particular sizes, doses or procedures. For example, syringes configured as disclosed here can also be configured for use in aspirating fluid samples from patients during clinical procedures such as knee or spinal taps. Because the overall syringe length is reduced through use of the disclosed frontal attachment in combination with the novel barrel, longer needles can be used and still be retracted into the syringe following use. As used in this disclosure, "retracted" or "retraction" refer to the process by which a needle is moved from a forwardly projecting use position to a post-use position in which the needle point no longer projects forwardly from the frontal attachment, no matter whether the force acting upon the needle is pushing or pulling the needle tip rearwardly from the forwardly projecting position.

Another arrangement of a syringe for medical use is disclosed having a barrel with a longitudinally extending tubular fluid chamber and at least one longitudinally extending, outwardly facing, substantially flat indicia display surface disposed proximally to the fluid chamber, a plunger slidably inserted into the fluid chamber, a needle projecting forwardly from the barrel in fluid communication with the fluid chamber; and a needle safety device of fixed, predetermined length. The needle safety device desirably includes an activation handle slidably engaging the barrel and a forwardly projecting needle tip shield encircling the needle. In a first stop position, the needle tip shield is disposed around a nose portion of the barrel. In a second stop position, the activation handle is moved forwardly under manual pressure following use of the syringe, causing the needle tip shield to move forwardly to circumferentially surround and cover a tip end of the needle.

Another arrangement is disclosed that desirably embodies one and preferably two oppositely facing, substantially flat indicia display surfaces and also embodies a needle safety device having a needle tip shield that extends circumferentially around the needle and is desirably coaxially aligned with the needle. The needle tip shield is attached or connected to, or unitarily molded together with, an activation handle that slidably engages the barrel. Suitable rails, ramps, stop shoulders and detents or other similarly effective means are desirably provided as part of the needle safety device and the barrel so that the activation handle can be advanced smoothly and without substantial interference when desired, and will not accidentally retract afterward to expose the needle tip. The needle safety device of this arrangement eliminates the need for having a needle retraction mechanism, a needle retraction cavity or any transverse sliding movement of the barrel relative to the needle to protect users from accidental needle sticks. Following use of the syringe, the needle tip shield is selectively advanced to protect the user from the forwardly projecting needle tip by applying manual pressure to a touch surface of the activation handle that is located rearwardly of the needle and needle tip shield. The activation handle slidably engages the syringe barrel and is forwardly slidable relative to the barrel from a first position, in which the needle tip is uncovered, to a second position in which the needle tip is surrounded and protected against inadvertent needle sticks by the needle tip shield. The needle tip is desirably disposed sufficiently inside the needle tip shield once the needle safety device is fully extended relative to the barrel that someone handling the used syringe will not inadvertently be subjected to a needle stick injury by simply placing a fingertip over the end of the syringe.

In another arrangement, the activation handle further comprises a manually engageable touch surface.

In another arrangement, the barrel and the activation handle comprise cooperatively engageable elements that enable manually actuated and controllable, longitudinally slidable movement of the activation handle relative to the barrel between two predetermined stop positions. The first stop position is a fully retracted use position in which the needle tip shield substantially surrounds the nose. The second stop position is a fully extended post-use position in which the needle tip shield circumferentially surrounds a tip end of the forwardly projecting needle.

In one preferred arrangement, the forwardly projecting needle is disposed in fixed relation to the nose of the barrel but it will be appreciated upon reading this disclosure that a similarly configured syringe can be made with needles configured to be selectively attachable to the barrel.

These and other features of the present invention will be better understood from a consideration of the following detailed description of various arrangements and appended claims in conjunction with the accompanying drawings.

### BRIEF DESCRIPTION OF THE DRAWINGS

The syringe is further described and explained in relation to the following drawings wherein:
FIG. 1 is a top front perspective view of one arrangement in which the needle cover (including the locking member) and the plunger end cap are installed in the position in which the subject syringes can be packaged, shipped and stored, with the locking member of the needle cover restricting lateral sliding movement of the barrel relative to the frontal attachment prior to use; The arrangement shown in Figure 1, and in Figures 2 to 13 is helpful for understanding the present invention.
FIG. 2 is a top front perspective view of the arrangement of FIG. 1 in which the needle cover is removed;
FIG. 3 is an exploded top front perspective view of the arrangement of FIG. 1;
FIG. 4 is a right side elevation view of the arrangement of FIG. 2;
FIG. 5 is a cross-sectional view taken along line 5-5 of FIG. 4;
FIG. 6 is an exploded detail perspective view, partially broken away, of the frontal attachment of the arrangement of FIG. 3 shown in juxtaposition to the front portion of the barrel of the arrangement of FIG. 3;
FIG. 7 is a top rear perspective view of the frontal attachment of FIG. 3;
FIG. 8 is a right side elevation view of the arrangement of FIG. 4 with the plunger cap removed and with the plunger withdrawn to an aspirated position;
FIG. 9 is a cross-sectional view taken along line 9-9 of FIG. 8;
FIG. 10 is a right side elevation view of the arrangement of FIG. 8 with the needle retracted and with the plunger fully advanced inside the barrel;
FIG. 11 is a cross-sectional view taken along line 11-11 of FIG. 10;
FIG. 12 is a front elevation view of the arrangement of FIG. 2;
FIG. 13 is a front elevation view of the arrangement of FIG. 12 with the frontal attachment repositioned relative to the barrel and with the needle retracted to the position shown in FIGS. 10 and 11;
FIG. 14 is a top front perspective view of another arrangement of a syringe, with both the needle cap and the plunger cap being shown in place as they would be when the syringe is first removed from its packaging; The arrangement shown in Figure 14, and the further Figures 15 to 24 is an embodiment of the present invention and covered by the present claims.
FIG. 15 is the syringe arrangement as shown in FIG. 14 with the needle cap and plunger cap having been removed;
FIG. 16 is the syringe arrangement as shown in FIG. 15 with the needle safety device moved forwardly relative to the barrel and needle, and with the needle tip shield surrounding and covering the needle tip;
FIG. 17 is an exploded top perspective view of the syringe arrangement of FIG. 14;
FIG. 18 is a side elevation view of the syringe arrangement of FIG. 14;
FIG. 19 is a top plan view of the syringe arrangement of FIG. 14;
FIG. 20 is a cross-sectional view taken along line 2-2- of FIG. 19;
FIG. 21 is the opposite side elevation view of the syringe arrangement as in FIG. 15 but rotated 180° around the longitudinal axis;
FIG. 22 is a side elevation view of the syringe arrangement as in FIG. 16;
FIG. 23 is a cross-sectional side elevation view taken along the longitudinal axis of the needle of the syringe arrangement as in FIG. 22; and
FIG. 24 is a bottom plan view of the syringe arrangement as in FIG. 18.

It should be noted that the drawings are not necessarily to scale.

### DESCRIPTION OF PREFERRED ARRANGEMENTS

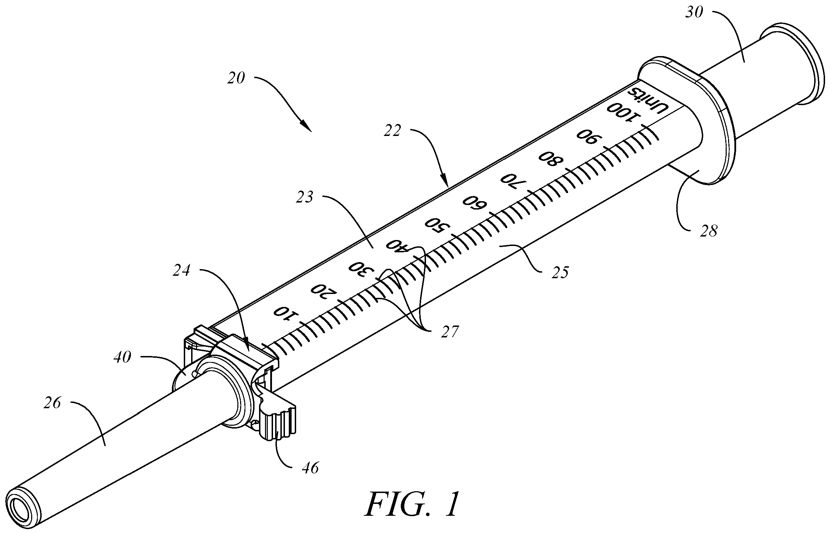

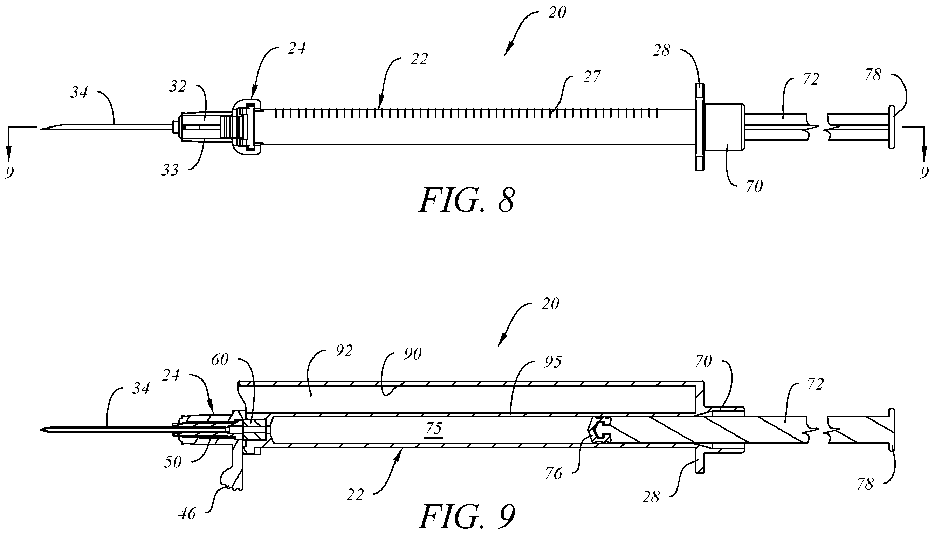

Referring to FIG. 1, syringe 20 comprises barrel 22 further comprising substantially flat display surface 23, frontal attachment 24, outside wall 25 of fluid chamber 75 (visible in FIG. 9), selectively removable needle cover 26, an array of volumetric measuring indicia 27, finger flange 28, removable plunger cap 30 and locking member 40. As shown in FIG. 1, the principal volumetric measuring indicia 27 are applied to display surface 23, including for example at least the Arabic numerals identifying the number of fluid units and the associated principal measurement indicia. It should be appreciated, however, that the accompanying figures of the drawings are for illustrative purposes and are not drawn to scale and that placement of some features such as the secondary unit markings 27 relative to substantially flat display surface 23 can vary from the positions shown. At least a portion of each secondary (individual) unit indicia or markings desirably commences on display surface 23 or proximal to the edge of display surface 23, and optionally continues for a short distanced onto the curved outer wall of fluid chamber 25. Such placement is understood to be within the operational capabilities of conventional pad printing technology that is now readily available to those of ordinary skill in the art.

As depicted in FIG. 1, syringe 20 has frontal attachment 24 shown in the "pre-use" configuration with needle cover 26 and locking member 40 in place to prevent frontal attachment 24 from shifting laterally relative to barrel 22 prior to use because of pressure inadvertently applied to textured touch pad 46 or to the opposite side of barrel 22. Removable plunger cap 30 is also installed behind finger flange 28 to prevent the plunger (not visible) from being accidentally withdrawn from barrel 22 and to prevent inadvertent contamination inside the rear opening of barrel 22 or around the rearwardly extending handle portion of the plunger, as seen in FIG. 9, which is discussed below.

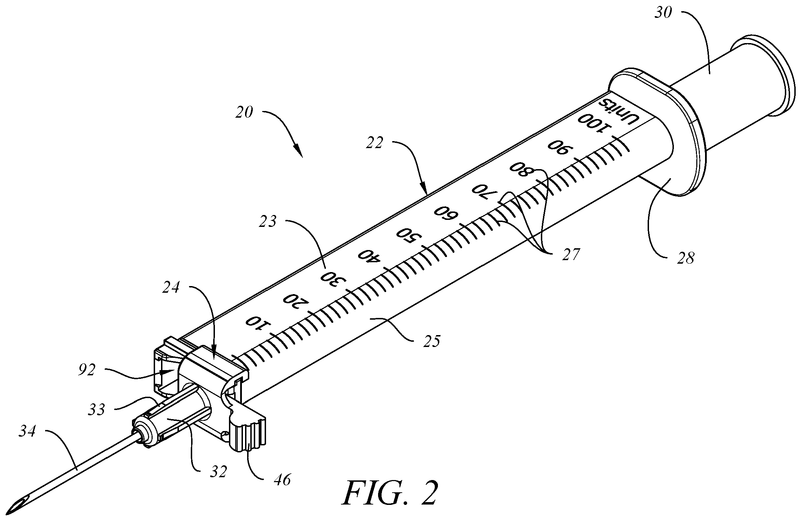

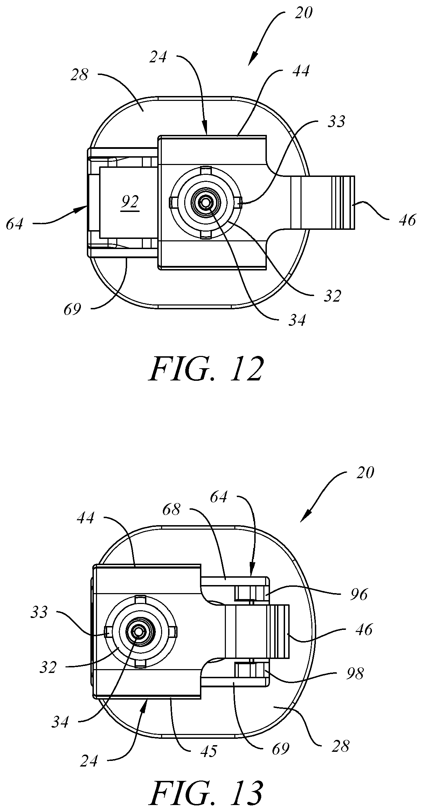

Referring to FIGS. 2, 4-5 and 12, barrel 22 and frontal attachment 24 of syringe 20 are shown in the same position as in FIG. 1 except that needle cover 26 (with locking member 40) is removed. The front opening into needle retraction cavity 92 is more clearly visible, and needle 34 is also visible, projecting forwardly from needle support member 32 of frontal attachment 24. Circumferentially spaced, axially tapered ribs 33 are disposed around needle support member 32 and provide surfaces for frictional engagement with the inside surface of needle cover 26 prior to removal. In FIG. 2, needle 34 is desirably coaxially aligned with the longitudinal axis through fluid chamber 75 (FIG. 9).

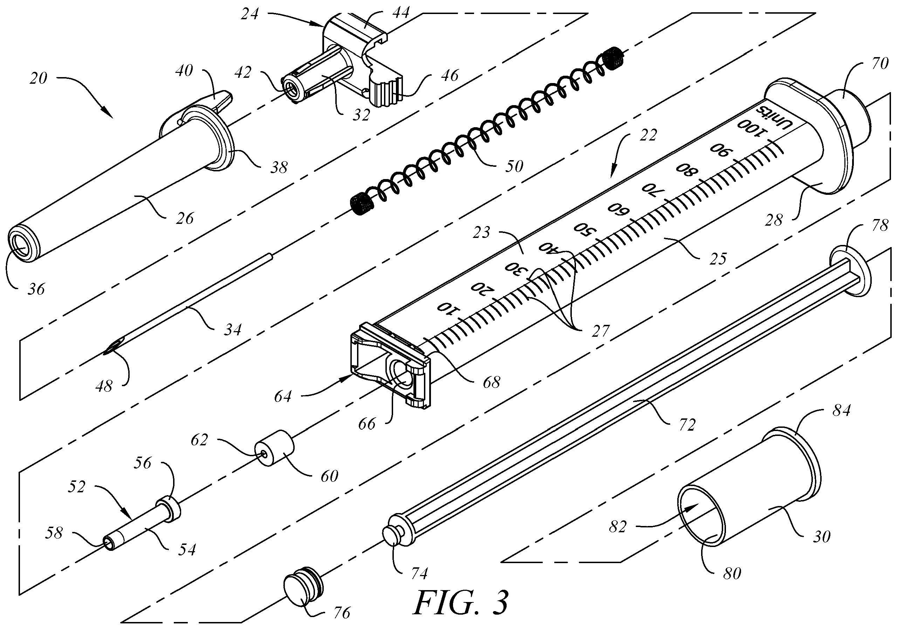

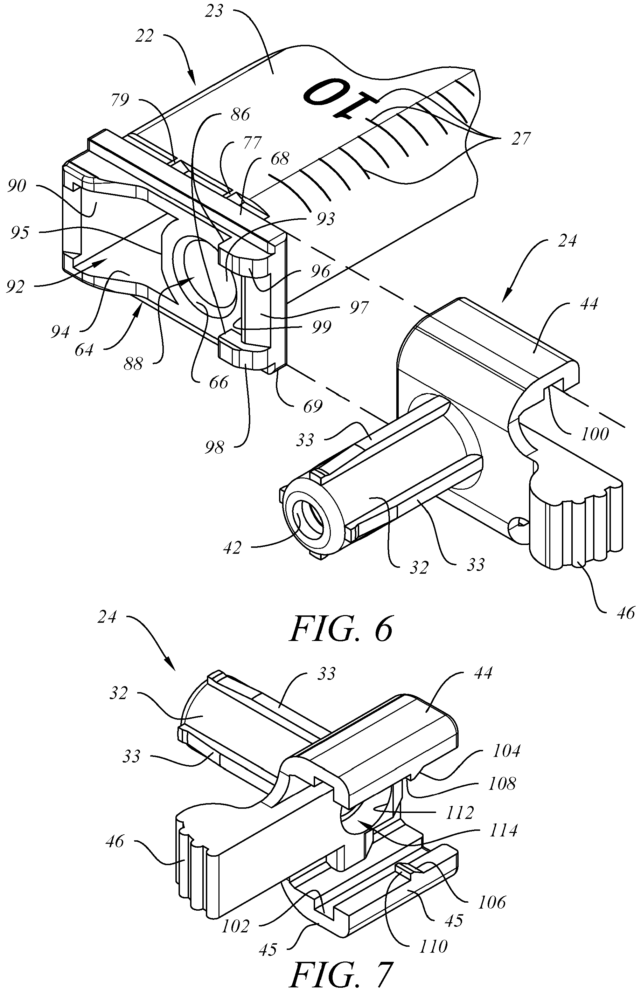

Referring to FIG. 3, needle cap 26 of syringe 20 further comprises a forwardly facing, inwardly tapered, substantially cylindrical sidewall having front end 36, rearwardly facing annular collar 38, and locking member 40 projecting rearwardly past annular collar 38. Frontal attachment 24 further comprises forwardly projecting, substantially tubular needle support member 32 with front opening 42, upper guide member 44, lower guide member 45 (visible in FIG. 7), and laterally projecting textured touch pad 46.

Syringe 20 desirably includes needle 34 with forwardly facing beveled needle tip 48, and a needle retraction mechanism further comprising coiled compression spring 50 and needle holder 52. Needle holder 52 further comprises an elongated shaft 54 with tubular bore 58 that is insertable into the rear of spring 50. The diameter of head 56 of needle holder 52 is sufficiently greater than the inside diameter of spring 50 that spring 50 can be held in compression by head 56 when frontal attachment 24 is slidably engaged with front portion 64 of barrel 22 as discussed in relation to FIGS. 6 and 7 below. The rear end of needle 34 is insertable into tubular bore 58 of needle holder 52 and attachable in fixed relation to the inside of elongated shaft 54 by any suitable, commercially available means such as an adhesive. Although a needle retraction mechanism as disclosed here is satisfactory for use in syringe 20, it will be appreciated that other similarly effective elements and mechanisms useful for rearwardly biasing needle 34 inside syringe 20 can also be used in making the invention.

Still referring to FIG. 3, annular polymeric fluid seal 60 with tubular bore 62 is desirably insertable into recess 66 in front portion 64 of barrel 22 so that the forwardly facing end of seal 60 is disposed in abutting contact with rearwardly facing head 56 of needle holder 52 when the needle retraction mechanism is installed inside frontal attachment 24 and frontal attachment 24 is attached in slidable engagement with front portion 64 of barrel 22. When frontal attachment 24 of syringe 20 is assembled to front portion 64 of barrel 22 during manufacture, a continuous, substantially linear, fluid flow path is established through needle 34, needle holder 52 and annular fluid seal 60 into tubular, longitudinally extending fluid chamber 75 (visible in FIG. 9).

In addition to front portion 64, barrel 22 further comprises substantially flat display surface 23, curved outside wall surface 25, finger flange 28 and rearwardly projecting annular collar 70. During assembly of syringe 20, elastomeric plunger seal 76 is desirably installed on forwardly projecting boss 74 on the front end of plunger handle 72 opposite rearwardly facing plunger thumb pad 78, and plunger handle 72 is then inserted into a rearwardly facing opening defined by annular collar 70. Assembly of syringe 20 is then completed by installing substantially cylindrical plunger cap 30 on the rearwardly facing end of barrel 22. Plunger cap 30 further comprises open front end 80, cylindrical bore 82 and closed rear end 84. Plunger cap 30 is installed around plunger thumb pad 78 and in frictional engagement with the outside wall of annular collar 70. Volumetric measuring indicia 27 applied as discussed in relation to FIG. 1 also appear on the outwardly facing portions of substantially flat display surface 23 and curved outside wall surface 25.

FIGS. 3, 6 and 11 further disclose the forwardly facing opening into needle retraction cavity 92 in relation to front portion 64 of barrel 22. Needle retraction cavity 92 has a closed rear end that is adjacent to finger flange 28, and is bounded by side walls 90, 95, bottom wall 94, and a top wall that also includes upwardly facing, substantially flat display surface 23 of barrel 22.

The assembly of frontal attachment 24 to front portion 64 of barrel 22 of syringe 20 is further described and explained in relation to FIGS. 6 and 7. Referring to FIG. 6, annular fluid seal 60 (visible in FIG. 3) is first inserted into recess 66 of front portion 64 of barrel 22. Front portion 64 further comprises laterally extending top rail 68 and bottom rail 69 that are disposed in transverse relation to the longitudinal axis through fluid chamber 75 (FIG. 9) of barrel 22. Rounded attachment guides 96, 98 are disposed forwardly of top and bottom rails 68, 69, respectively, and are configured to facilitate the assembly of frontal attachment 24 to front portion 64 of barrel 22 by passing above and below the arm connecting textured touch pad 46 to the back side of frontal attachment 24.

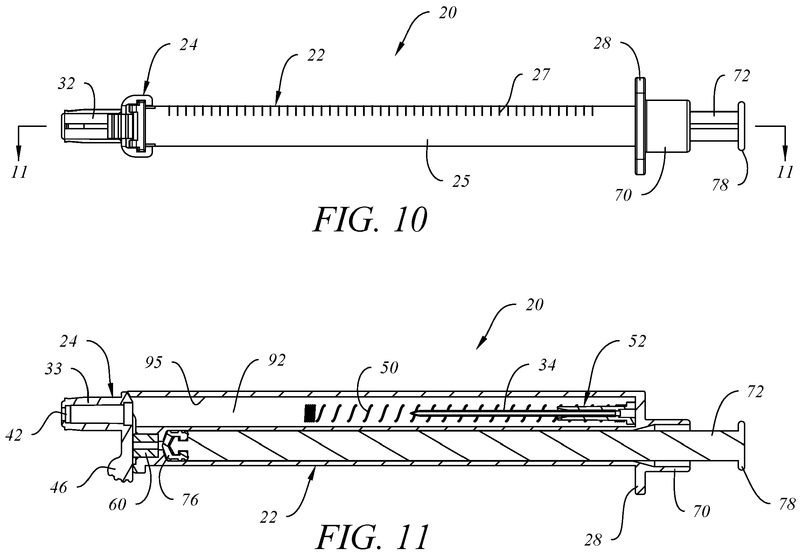

Referring to FIG. 7, annular opening 112 is provided in the back side of frontal attachment 24 to facilitate insertion of spring 50 and needle holder 52, discussed above in relation to FIG. 3, and spring 50 is desirably compressed between an annular shoulder inside front opening 42 (visible in FIG. 11) and is held in compression behind head 56 of needle holder 52 while frontal attachment 24 is moved into sliding engagement with front portion 64 of barrel 22. Frontal attachment 24 further comprises upper guide 44 having a downwardly facing, laterally extending recess 100 and lower guide 45 having an upwardly facing, laterally extending recess 102. Upper ramp and lower ramp 104, 106, respectively, of frontal attachment 24 are configured to slide over two laterally spaced-apart sets of opposed ramps with blocking shoulders 77, 79 (the lower set not being visible in FIG. 6). Laterally extending top rail 68 and bottom rail 69 of front portion 64 of barrel 22 are desirably cooperatively engaged with upper and lower recesses 100, 102, respectively, by sliding frontal attachment 24 onto front portion 64 of barrel 22 when frontal attachment 24 and front portion 64 are positioned as shown in FIG. 6. As pressure is applied to textured touch surface 46, frontal attachment 24 moves along rails 68, 69 until top and bottom blocking shoulders 108 of upper guide 44 pass over and then drops into facing relation to upper and lower blocking shoulders 77. At this time, needle support member 32 and needle holder 52 (not shown in FIGS. 6 and 7) are desirably coaxially aligned with the longitudinal axis of fluid chamber 75 (visible in FIG. 9). Any attempt to move frontal attachment 24 back to a disconnected position as shown in FIG. 6 will be resisted by facing and abutting contact between upper and lower blocking shoulders 77 of front portion 64 and top and bottom blocking shoulders 108, 110, respectively, of frontal attachment 24.

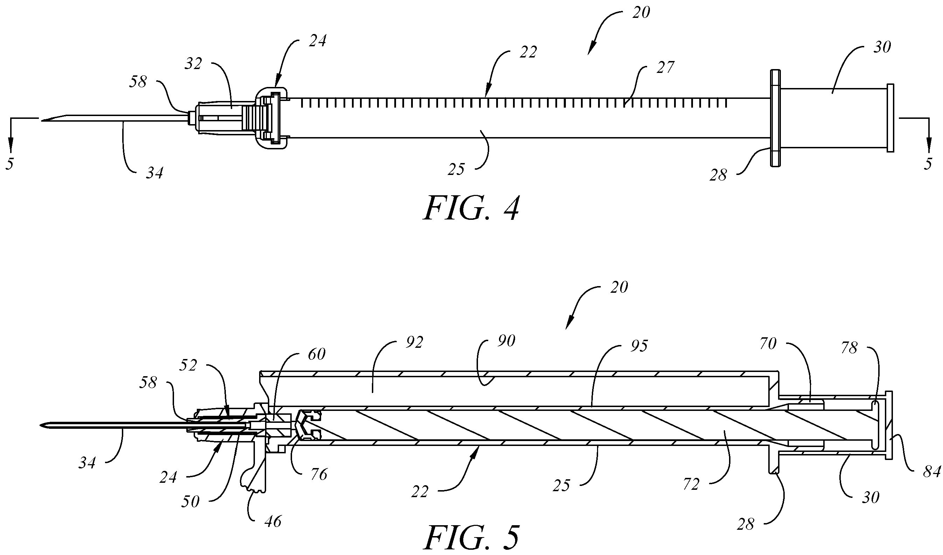

FIGS. 4 and 5 depict syringe 20 of FIG. 2 with the needle cover removed and with plunger cap 30 still in place and with textured touch surface 46 in the initial position relative to barrel 22 as described above. Needle 34 is installed inside bore 58 of needle holder 52, which is seated inside needle support member 32 of frontal attachment 24, with spring 50 compressed between head 56 (FIG. 3) of needle holder 52. Spring 50 applies a rearwardly directed biasing force to needle holder 52 and needle 34, and is pressed by spring 50 into facing and abutting contact with the front surface of annular fluid seal 60, thereby establishing a coaxially aligned fluid path through needle 34, needle holder 52 and fluid seal 60 into fluid chamber 75 (visible in FIG. 9) of barrel 22. As shown in FIGS. 4 and 5, plunger seal 76 is pushed fully forward into substantially cylindrical fluid chamber 75 of barrel 22.

Referring to FIGS. 8-9, syringe 20 is configured so that fluid can be aspirated into the syringe, with textured touch pad 46 again in the same initial position as previously described. Plunger cap 30 (as seen in FIGS. 2, 4-5) is removed and plunger handle 72 is withdrawn as it would be while aspirating fluid into fluid chamber 75. In FIG. 9 it is seen that needle retraction cavity 92 is substantially coextensive in length with fluid chamber 75, thereby facilitating the placement of volumetric measuring indicia 27 on substantially flat surface 23 (visible in FIG. 3) that facilitates full utilization of the volume of fluid chamber 75 and also facilitating the retraction of needles such as biopsy needles that are often substantially longer than needle 34 (visible inside needle retraction cavity 92 in FIG. 11 below). Once fluid is aspirated into fluid chamber 75, fluid can also be injected into a patient or expelled from fluid chamber 75 by pressing forwardly on plunger thumb pad 78 while applying finger pressure against the forwardly facing side of flange 28.

Referring to FIGS. 10-11 and 13, plunger handle 72 and plunger seal 76 have been pushed forwardly to empty fluid chamber 75. To initiate needle retraction, pressure is applied to textured touch pad 46, which moves frontal attachment to a position where needle support member 32 is aligned with needle retraction cavity 92. Because needle retraction chamber 92 has a front opening that is larger than head 56 of repositioned needle holder 52, the biasing force of compressed spring 52 pushes needle holder 52 rearwardly and causes needle holder 52 and needle 34 to be propelled into the distal end of needle retraction cavity 92.

Referring again to FIGS. 6 and 7, the further application of force to textured touch surface 46 of frontal attachment 24 relative to barrel 22 as described above in relation to FIGS. 10-11 produces sliding relative movement between frontal attachment 24 and front portion 64 of barrel 22. This movement causes upper ramp 104 and lower ramp 106 to slide over the second set of opposed ramps and blocking shoulders 79 so that top and bottom blocking shoulders 108, 110 are placed in facing and abutting contact with upper and lower blocking shoulders 79, thereby preventing frontal attachment 24 from being returned to the use position relative to barrel 22.

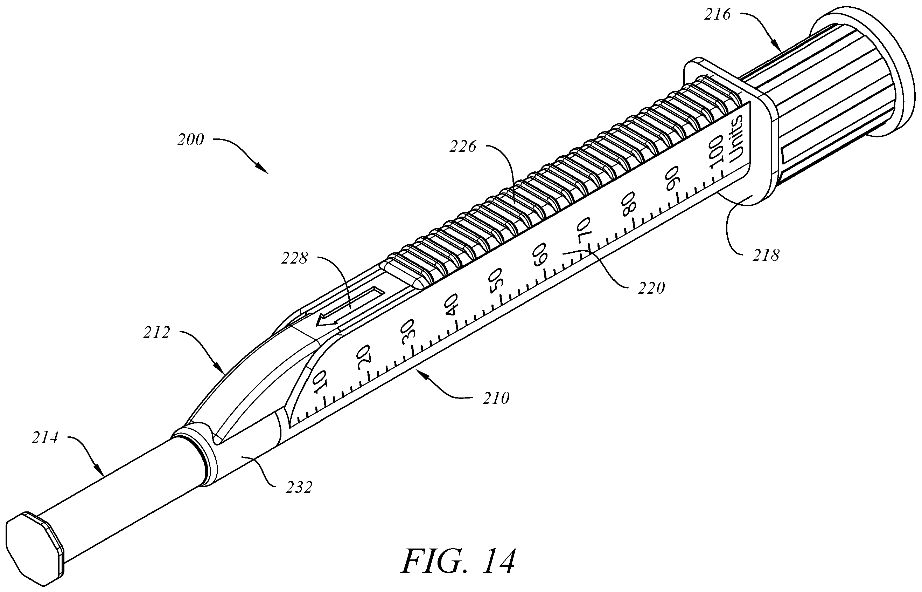

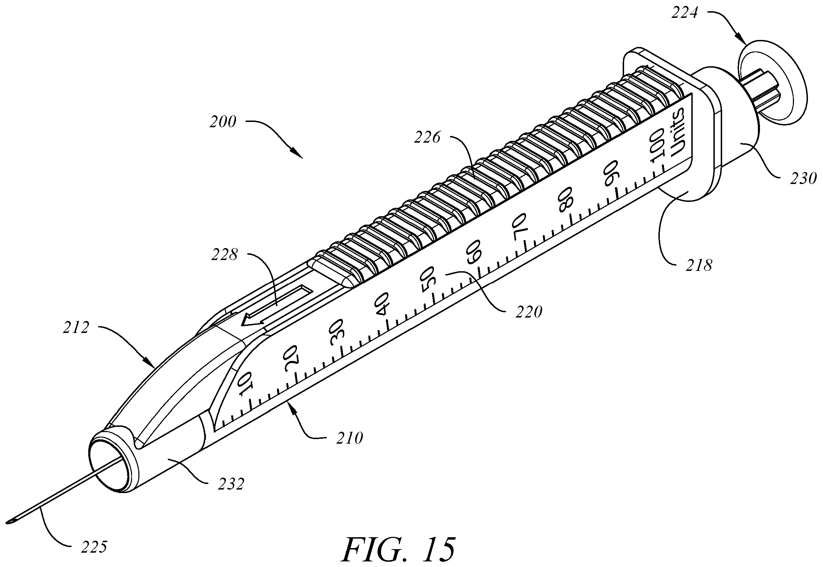

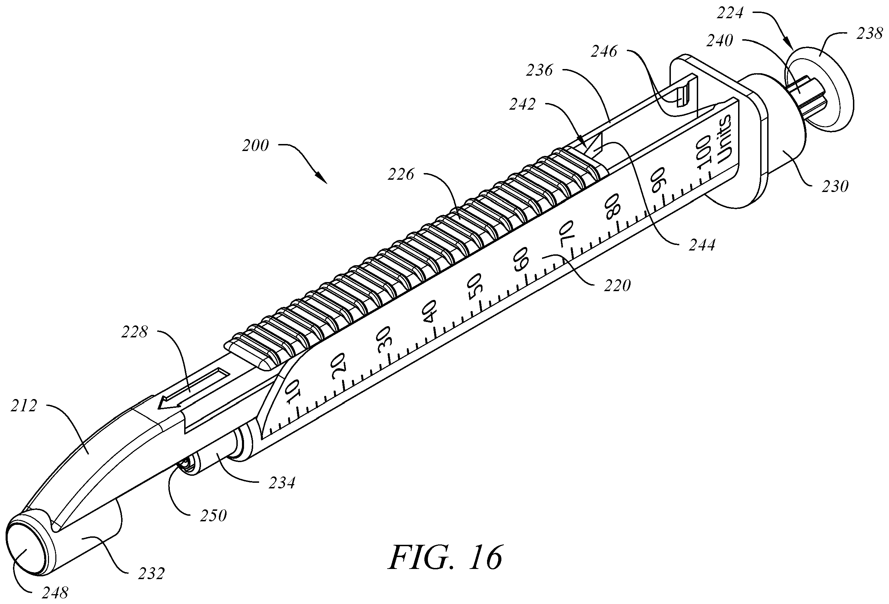

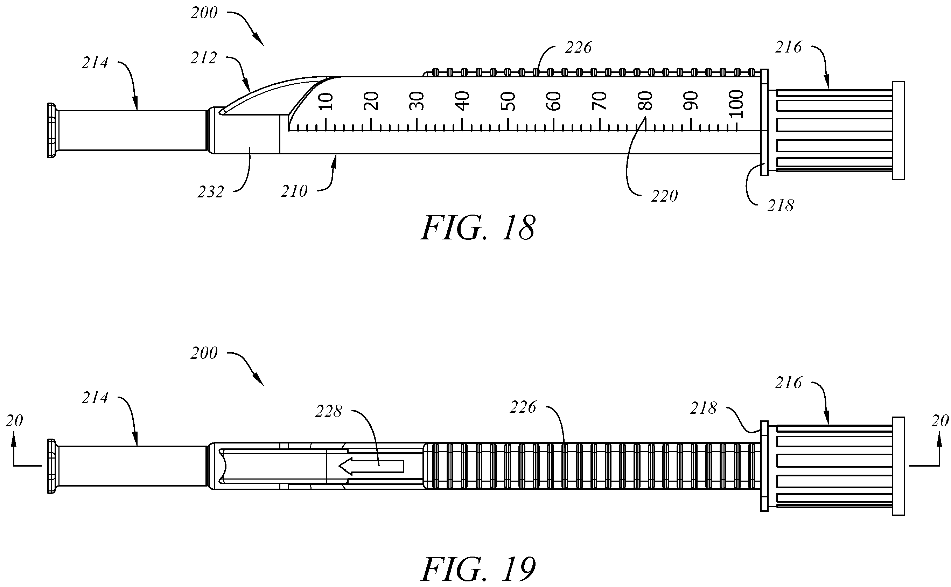

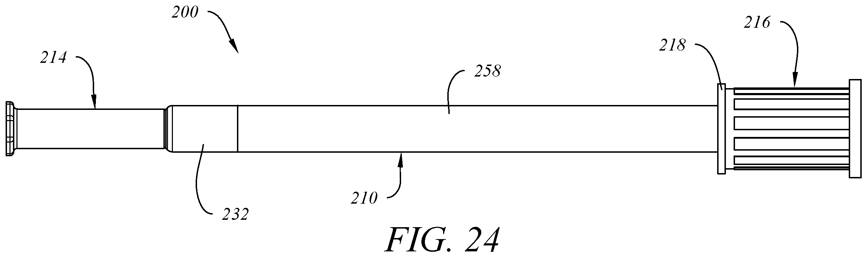

Referring to FIGS. 14-24 generally, and specifically to FIGS. 14-17, another arrangement is disclosed comprising syringe 200 further comprising barrel 210, needle safety device 212, needle cap 214 and plunger cap 216. Barrel 210 desirably comprises at least one longitudinally extending, substantially flat indicia display surface 220 marked with an easily readable volumetric scale. In one preferred arrangement, barrel 210 comprises two oppositely facing, substantially flat indicia display surfaces 220, 236 (FIG. 16). Following removal of needle cap 214 and plunger cap 216 (FIG. 14) from the device, forwardly projecting needle 225 and rearwardly projecting plunger 224 (FIG. 15) become visible. Needle 225 is coaxially aligned with plunger 224 and is held in fixed relation to a narrow bore 250 inside nose 234 (FIGS. 16, 17, 20) at the front end of barrel 210. (Alternatively, it will be appreciated that needle 225 and nose 234 can be cooperatively configured so that needle 225 is releasably attachable to nose 234 if desired to permit the use of differently sized needles with barrel 234.)

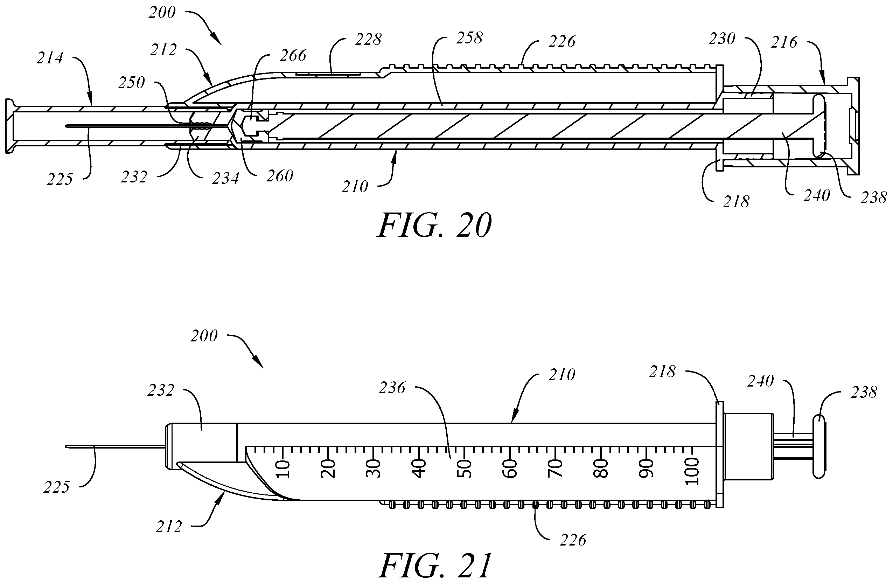

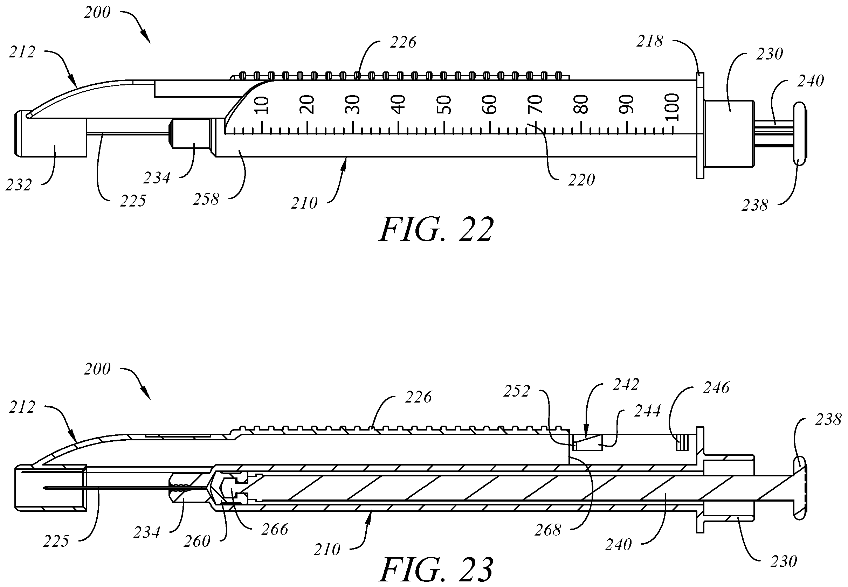

Bore 250 places needle 225 in fluid communication with a fluid reservoir disposed inside tubular barrel 258 between nose 234 and plunger seal 260 on the front end of plunger handle 240. When plunger handle 240 is pushed fully forward against the rear of nose 234 of barrel 210 prior to an injection (FIG. 20) or following an injection (FIG. 23) the fluid chamber is not visible. When syringe 200 is prepared for an injection, as discussed below, the fluid reservoir is the space (not visible in these views) inside tubular section 258 that is between the rear of nose 234 and the front of plunger seal 260 as fluid is drawn into syringe 200.

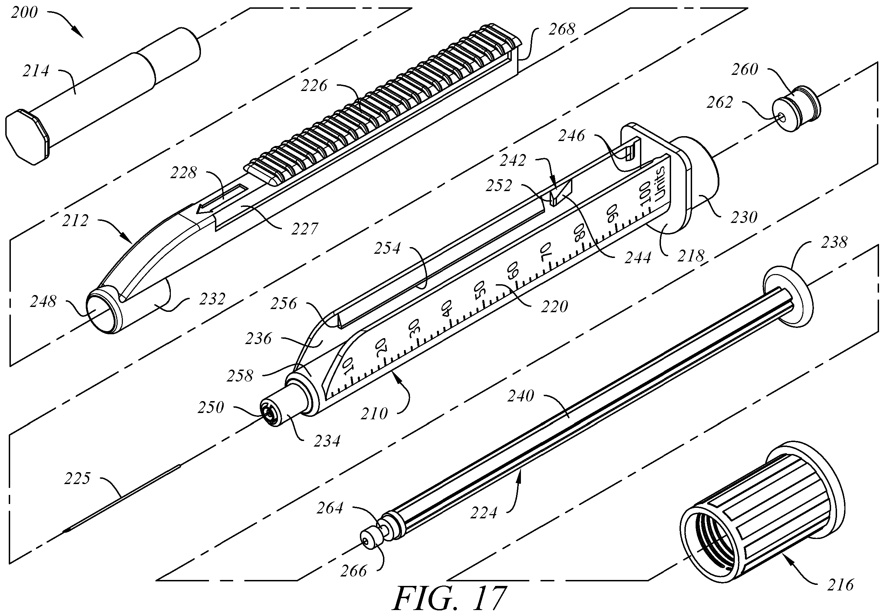

Referring to FIG. 17, needle safety device 212 also comprises activation handle 228 and needle tip shield 232. Activation handle 228 further comprises rear end 268, touch pad 226 and a pair of longitudinally extending channels 227 disposed on opposite sides of activation handle 228. The function of channels 227 is discussed below in relation to sliding engagement with barrel 210. Needle tip shield 232 further comprises inside bore 248 that is coaxially aligned with needle 225 and with nose 234 of barrel 210. Needle tip shield 232 desirably has an inside diameter that will allow needle tip shield 232 to slide over and surround coaxially aligned nose 234 when in the position shown in FIGS. 15, 21 and needle tip shield 232 is sufficiently long to safely cover the tip end of needle 225 when moved forwardly as in FIGS. 16, 22-23. Slight frictional engagement can be provided between the inside wall of needle tip shield 232 and nose 234 to help hold needle safety device 212 in a stable axial position relative to barrel 210 during use of syringe 200. Needle safety device 212 is desirably unitarily molded from a polymeric material and is cooperatively sized and configured to slidably engage barrel 210, also taking into consideration the range of needle lengths intended for use with syringe 200.

Referring again to FIG. 17, barrel 210 further comprises coaxially aligned nose 234 with internal bore 250 and tubular section 258 extending rearwardly from nose 234 to fingertip flange 218. The rear end of tubular section 258 is open and communicates with the inside of cylindrical, rearwardly extending collar 230 to which plunger cap 216 is releasably attachable. Opposed indicia display surfaces 220, 236 (FIG. 16) are desirably parallel, longitudinally extending, substantially flat, printable surfaces each having an oppositely facing volumetric scale comprising appropriate indicia located adjacent to tubular section 258 to facilitate easy reading of a liquid level inside tubular section 258 by a user. A longitudinally extending rail 256 with an inwardly projecting retainer edge 254 is desirably provided on the inwardly facing walls of each of the outwardly facing indicia display surfaces 220, 236. Longitudinally extending rails 256 are cooperatively sized and configured to engage and provide a smooth, slidable interface along each of aligned channels 227 of needle safety device 212. Barrel 210 is desirably molded from a medical grade polymeric material and is sufficiently transparent to permit the liquid level drawn into tubular section 258 of syringe 200 to be plainly viewed by a user. Still referring to FIG. 17, plunger 224 further comprises thumb cap 238, plunger handle 240, plunger seal retainer body 266 and annular recess 264.

The cooperatively configured structural elements and operation of needle safety device 212 relative to barrel 210 of syringe 200 are further described and explained in relation to FIGS. 15-17, 20, 22-23. During assembly of syringe 200, activation handle 228 of needle safety device 212 is aligned with and inserted over tubular section 258 of barrel 210 with rails 256 engaging channels 227. Needle safety device 212 is moved rearwardly relative to barrel 210 until needle tip shield 232 surrounds nose 234 of barrel 210 and rear end 268 of needle safety device 212 frictionally engages oppositely disposed rear stops 246. Slide stops 242 each having a ramp 244 and stop shoulder 252 are provided rearwardly of rails 256 and forwardly of rear stops 246. Opposed and facing ramps 244 allow activation handle 228 to slide forwardly relative to barrel 210 to a point where needle tip shield 232 covers and protects the tip end of needle 225 following use of syringe 210. After the tip end of needle 225 is covered, opposed and facing stop shoulders 252 prevent activation handle 228 from being moved rearwardly relative to barrel 210 to again expose the needle tip.

When needle safety device 212 and needle tip shield 232 are disposed in the position shown in FIGS. 15, 20-21 (referred to as the "first stop position") relative to barrel 210 and needle 225, needle tip shield 232 surrounds nose 234 of barrel and a portion of needle 225. When needle safety device 212 of syringe 200 is in the first stop position, the front tip of needle 225 can be inserted through the stopper of a medicine vial (e.g., an insulin bottle) and medicine can be drawn into a fluid reservoir disposed inside tubular section 258 (FIGS. 17, 20) of barrel 210 by pulling rearwardly on plunger 224 that slidably engages the inside wall of tubular section 258.

Following an injection or other use of syringe 200, syringe 200 can be reconfigured into a "safe" position with the needle tip covered and protected from inadvertent contact with a health care professional or patient by selectively moving needle safety device 212 forwardly to the position shown in FIGS. 16, 22-23, which is the "second stop position." When syringe 200 is configured with needle safety device 212 in the second stop position, the front tip of needle 225 is circumferentially surrounded by needle tip shield 232 and (as shown in FIGS. 16, 23) the front end of needle tip shield 232 extends sufficiently forward of the tip end of needle 225 that an individual will not receive an unintended needle stick if the front end of syringe 200 is pressed against his or her skin.

## Claims

1. A syringe (200) for medical use, comprising:
a barrel (210) comprising:
a fluid chamber (75) comprising a tubular sidewall extending longitudinally,
a nose (234) disposed forwardly of the fluid chamber,
a rearwardly facing opening, and
two oppositely facing, substantially flat, parallel indicia display surfaces (220, 236) extending longitudinally along the tubular sidewall and disposed in fixed relation to the fluid chamber (75),
each of the indicia display surfaces (220, 236) comprising a longitudinally extending rail (256) projecting inwardly from an inwardly facing wall and volumetric dosage scale indicia disposed on an outwardly facing wall to facilitate reading of a liquid level inside the fluid chamber (75) through at least a portion of the tubular sidewall;
a plunger (224) slidably inserted into the fluid chamber (75) through the rearwardly facing opening;
a needle (225) projecting forwardly from the nose (234) in fluid communication with the fluid chamber (75); and
a needle safety device (212) comprising a needle tip shield (232), an activation handle (228) disposed rearwardly of the needle tip shield (232), and a longitudinally extending channel (227) disposed on each side of the activation handle (228);
wherein the longitudinally extending channels (227) are cooperatively sized and configured to engage and provide a slidable interface with the longitudinally extending rails (256) of the indicia display surfaces (220, 236) to allow the needle safety device (212) to slide between a first stop position and a second stop position relative to the barrel (210);
wherein the needle tip shield (232) circumferentially surrounds the nose (234) of the barrel (210) when the needle safety device (212) is disposed in the first stop position; and
wherein the needle tip shield (232) circumferentially surrounds a front tip of the needle (225) when the needle safety device (212) is moved forwardly from the first stop position to the second stop position.

2. The syringe (200) of claim 1, wherein each of the longitudinally extending rails (256) of the indicia display surfaces (220, 236) comprises an inwardly projecting retainer edge (254).

3. The syringe (200) of claim 2, wherein the longitudinally extending channels are (227) cooperatively sized and configured to engage and provide a slidable interface with the inwardly projecting retainer edges (254) of the longitudinally extending rails (256) of the indicia display surfaces (220, 236).

4. The syringe of claim 3, wherein opposed and facing stop shoulders (252) prevent the needle safety device (212) from being moved rearwardly relative to the barrel to again expose the front tip of the needle (225) after the front tip of the needle (225) and is covered by the needle tip shield (232) when the needle safety device (21) is in the second stop position.

5. The syringe (200) of claim 1, wherein the volumetric dosage scale indicia is applied to the indicia display surface by pad printing.

6. The syringe (200) of claim 1, wherein the volumetric dosage scale indicia is applied to the outwardly facing walls of the indicia display surfaces (220, 236) by at least one of a group of application methods consisting of molding, stamping, embossing, or printing,, wherein in particular the volumetric dosage scale indicia is applied to the indicia display surface (220, 236) by pad printing.

7. The syringe (200) of claim 1, wherein the two oppositely facing, substantially flat, parallel indicia display surfaces (220, 236) comprise at least one first slide stop disposed rearwardly of the longitudinally extending rails (256) and a second slide stop disposed forwardly of the first slide stop, wherein in particular a rear end of the needle safety device (212) engages the first slide stop in the first stop position, and wherein the rear end of the needle safety device (212) engages the second slide stop in the second stop position to prevent subsequent rearward movement of the needle safety device (212) from the second stop position towards the first stop position.

8. The syringe (200) of claim 1, wherein the activation handle (228) further comprises a touch pad (226).

9. The syringe (200) of claim 1, wherein the first stop position is a fully retracted use position in which the needle tip shield (232) substantially surrounds the nose (234) and the front tip of the needle (225) is exposed, wherein in particular the second stop position is a fully extended post-use position in which the needle tip shield (232) circumferentially surrounds and shields the front tip of the needle (225).

10. The syringe (200) of claim 1, wherein the needle (225) is disposed in fixed relation to the nose (234) of the barrel (210).

11. The syringe (200) of claim 10, further comprising: a removable needle cap (214) configured to cover the front tip of the needle (225) when the needle safety device (212) is in the first stop position and the removable needle cap (214) is disposed about the nose (234) of the barrel (210).

12. The syringe (200) of claim 1, wherein the barrel (210) comprises a rearwardly facing collar (230) sized and configured to engage and support a removable plunger cap (216), wherein in particular the barrel (210) comprises a transversely projecting finger flange (218) disposed forwardly of the rearwardly facing collar (230).

13. The syringe (200) of claim 1, wherein the plunger (224) comprises a plunger seal (260) that engages the tubular sidewall of the fluid chamber.

14. The syringe (200) of claim 1, wherein the volumetric dosage scale indicia laterally overlap at least a portion of the tubular sidewall of the fluid chamber, wherein in particular the volumetric scale indicia are disposed adjacent to the portion of the tubular sidewall of the fluid chamber such that the liquid level through the portion of the tubular sidewall and the volumetric scale indicia are visible simultaneously.

## Patentansprüche

1. Spritze (200) für medizinische Verwendung, umfassend:
einen Zylinder (210), umfassend:
eine Flüssigkeitskammer (75) mit einer sich in Längsrichtung erstreckenden röhrenförmigen Seitenwand,
eine vor der Flüssigkeitskammer angeordnete Nase (234),
eine nach hinten gerichtete Öffnung und
zwei einander gegenüberliegende, im Wesentlichen flache, parallele Markierungsanzeigeflächen (220, 236), die sich in Längsrichtung entlang der röhrenförmigen Seitenwand erstrecken und in fester Beziehung zur Flüssigkeitskammer (75) angeordnet sind,
wobei jede der Markierungsanzeigeflächen (220, 236) eine sich in Längsrichtung erstreckende Schiene (256), die von einer nach innen gerichteten Wand nach innen vorsteht, und eine Volumendosierungsskalenmarkierung umfasst, die auf einer nach außen gerichteten Wand angeordnet ist, um das Ablesen eines Flüssigkeitsstands innerhalb der Flüssigkeitskammer (75) durch mindestens einen Abschnitt der röhrenförmigen Seitenwand zu erleichtern;
einen Kolben (224), der durch die nach hinten gerichtete Öffnung in die Flüssigkeitskammer (75) verschiebbar eingesetzt ist;
eine Nadel (225), die von der Nase (234) nach vorne vorsteht und in Fluidverbindung mit der Flüssigkeitskammer (75) steht; und
eine Nadel-Sicherheitsvorrichtung (212), die eine Nadelspitzenabdeckung (232), einen hinter der Nadelspitzenabdeckung (232) angeordneten Aktivierungsgriff (228) und einen sich in Längsrichtung erstreckenden Kanal (227) aufweist, der auf jeder Seite des Aktivierungsgriffs (228) angeordnet ist;
wobei die sich in Längsrichtung erstreckenden Kanäle (227) zusammenwirkend dimensioniert und konfiguriert sind, um mit den sich in Längsrichtung erstreckenden Schienen (256) der Markierungsanzeigeflächen (220, 236) in Eingriff zu kommen und eine gleitende Schnittstelle mit diesen zu bilden, damit die Nadel-Sicherheitsvorrichtung (212) zwischen einer ersten Anschlagposition und einer zweiten Anschlagposition relativ zum Zylinder (210) gleiten kann;
wobei die Nadelspitzenabdeckung (232) die Nase (234) des Zylinders (210) umgibt, wenn die Nadel-Sicherheitsvorrichtung (212) in der ersten Anschlagposition angeordnet ist; und
wobei die Nadelspitzenabdeckung (232) eine vordere Spitze der Nadel (225) umgibt, wenn die Nadel-Sicherheitsvorrichtung (212) von der ersten Anschlagposition in die zweite Anschlagposition vorwärts bewegt wird.

2. Die Spritze (200) nach Anspruch 1, wobei jede der sich in Längsrichtung erstreckenden Schienen (256) der Markierungsanzeigeflächen (220, 236) eine nach innen vorstehende Rückhaltekanten (254) aufweist.

3. Die Spritze (200) nach Anspruch 2, wobei die sich in Längsrichtung erstreckenden Kanäle (227) zusammenwirkend dimensioniert und konfiguriert sind, um mit den nach innen vorstehenden Rückhaltekanten (254) der sich in Längsrichtung erstreckenden Schienen (256) der Markierungsanzeigeflächen (220, 236) in Eingriff zu kommen und eine gleitfähige Schnittstelle zu bilden.

4. Die Spritze nach Anspruch 3, wobei gegenüberliegende und einander zugewandte Anschlagschultern (252) verhindern, dass die Nadel-Sicherheitsvorrichtung (212) relativ zum Zylinder nach hinten bewegt wird, um die vordere Spitze der Nadel (225) wieder freizulegen, nachdem die vordere Spitze der Nadel (225) freigelegt wurde, und wobei die Nadel-Sicherheitsvorrichtung (21) von der Nadelspitzenabdeckung (232) abgedeckt ist, wenn sich die Nadel-Sicherheitsvorrichtung (21) in der zweiten Anschlagposition befindet.

5. Die Spritze (200) nach Anspruch 1, wobei die Volumendosierungsskala durch Tampondruck auf die Markierungsanzeigefläche aufgebracht ist.

6. Die Spritze (200) nach Anspruch 1, wobei die Volumendosierungsskala durch mindestens eines aus einer Gruppe von Aufbringungsverfahren, bestehend aus Formen, Stanzen, Prägen oder Drucken, wobei insbesondere die Volumendosierungsskalenmarkierung durch Tampondruck auf die Markierungsanzeigefläche (220, 236) aufgebracht ist.

7. Die Spritze (200) nach Anspruch 1, wobei die beiden einander gegenüberliegenden, im Wesentlichen flachen, parallelen Markierungsanzeigeflächen (220, 236) mindestens einen ersten Gleitanschlag, der hinter den sich in Längsrichtung erstreckenden Schienen (256) angeordnet ist, und einen zweiten Gleitanschlag, der vor dem ersten Gleitanschlag angeordnet ist, umfassen, wobei insbesondere ein hinteres Ende der Nadel-Sicherheitsvorrichtung (212) in der ersten Anschlagposition in den ersten Gleitschalter eingreift und wobei das hintere Ende der Nadel-Sicherheitsvorrichtung (212) in der zweiten Anschlagposition in den zweiten Gleitschalter eingreift, um eine nachfolgende Rückwärtsbewegung der Nadel-Sicherheitsvorrichtung (212) aus der zweiten Anschlagposition in Richtung der ersten Anschlagposition zu verhindern.

8. Die Spritze (200) nach Anspruch 1, wobei der Aktivierungsgriff (228) ferner ein Touchpad (226) umfasst.

9. Die Spritze (200) nach Anspruch 1, wobei die erste Stoppposition eine vollständig zurückgezogene Gebrauchsposition ist, in der die Nadelspitzenabdeckung (232) die Nase (234) im Wesentlichen umgibt und die vordere Spitze der Nadel (225) freiliegt, wobei insbesondere die zweite Stoppposition eine vollständig ausgefahrene Position nach der Verwendung ist, in der die Nadelspitzenabdeckung (232) die vordere Spitze der Nadel (225) umgibt und abschirmt.

10. Die Spritze (200) nach Anspruch 1, wobei die Nadel (225) in fester Beziehung zur Nase (234) des Zylinders (210) angeordnet ist.

11. Die Spritze (200) nach Anspruch 10, ferner umfassend: eine abnehmbare Nadelkappe (214), die so konfiguriert ist, dass sie die vordere Spitze der Nadel (225) abdeckt, wenn sich die Nadel-Sicherheitsvorrichtung (212) in der ersten Anschlagposition befindet und die abnehmbare Nadelkappe (214) um die Nase (234) des Zylinders (210) angeordnet ist.

12. Die Spritze (200) nach Anspruch 1, wobei der Zylinder (210) einen nach hinten gerichteten Kragen (230) umfasst, der so dimensioniert und konfiguriert ist, dass er mit einer abnehmbaren Kolbenkappe (216) in Eingriff kommt und diese stützt, wobei insbesondere der Zylinder (210) einen quer vorstehenden Fingerflansch (218) umfasst, der vor dem nach hinten gerichteten Kragen (230) angeordnet ist.

13. Die Spritze (200) nach Anspruch 1, wobei der Kolben (224) eine Kolbendichtung (260) umfasst, die in die röhrenförmige Seitenwand der Flüssigkeitskammer eingreift.

14. Die Spritze (200) nach Anspruch 1, wobei die volumetrischen Dosierungsskalenmarkierungen seitlich mindestens einen Teil der röhrenförmigen Seitenwand der Flüssigkeitskammer überlappen, wobei insbesondere die volumetrischen Skalenmarkierungen benachbart zu dem Teil der röhrenförmigen Seitenwand der Flüssigkeitskammer angeordnet sind, so dass der Flüssigkeitsstand durch den Teil der röhrenförmigen Seitenwand und die volumetrischen Skalenmarkierungen gleichzeitig sichtbar sind.

## Revendications

1. Seringue (200) à usage médical, comprenant :
un cylindre (210) comprenant :
une chambre de fluide (75) comprenant une paroi latérale tubulaire s'étendant longitudinalement,
un nez (234) disposé vers l'avant de la chambre de fluide,
une ouverture faisant face vers l'arrière, et
deux surfaces d'affichage d'indices (220, 236) parallèles, sensiblement plates, se faisant face de manière opposée, s'étendant longitudinalement le long de la paroi latérale tubulaire et disposées en relation fixe par rapport à la chambre de fluide (75),
chacune des surfaces d'affichage d'indices (220, 236) comprenant un rail s'étendant longitudinalement (256) se projetant vers l'intérieur depuis une paroi faisant face vers l'intérieur et des indices d'échelle de dosage volumétrique disposés sur une paroi faisant face vers l'extérieur pour faciliter une lecture d'un niveau de liquide à l'intérieur de la chambre de fluide (75) à travers au moins une portion de la paroi latérale tubulaire ;
un piston (224) inséré de manière à pouvoir coulisser dans la chambre de fluide (75) à travers l'ouverture faisant face vers l'arrière ;
une aiguille (225) se projetant vers l'avant depuis le nez (234) en communication fluidique avec la chambre de fluide (75) ; et
un dispositif de sécurité d'aiguille (212) comprenant une protection de pointe d'aiguille (232), un manche d'activation (228) disposé vers l'arrière de la protection de pointe d'aiguille (232), et un canal s'étendant longitudinalement (227) disposé sur chaque côté du manche d'activation (228) ;
dans laquelle les canaux s'étendant longitudinalement (227) sont calibrés de manière à pouvoir coopérer et configurés pour engager et fournir une interface pouvant coulisser avec les rails s'étendant longitudinalement (256) des surfaces d'affichage d'indices (220, 236) pour permettre au dispositif de sécurité d'aiguille (212) de coulisser entre une première position d'arrêt et une seconde position d'arrêt par rapport au cylindre (210) ;
dans laquelle la protection de pointe d'aiguille (232) entoure circonférentiellement le nez (234) du cylindre (210) lorsque le dispositif de sécurité d'aiguille (212) est disposé dans la première position d'arrêt ; et
dans laquelle la protection de pointe d'aiguille (232) entoure circonférentiellement une pointe avant de l'aiguille (225) lorsque le dispositif de sécurité d'aiguille (212) est déplacé vers l'avant depuis la première position d'arrêt vers la seconde position d'arrêt.

2. Seringue (200) selon la revendication 1, dans laquelle chacun des rails s'étendant longitudinalement (256) des surfaces d'affichage d'indices (220, 236) comprend un bord de retenue se projetant vers l'intérieur (254).

3. Seringue (200) selon la revendication 2, dans laquelle les canaux s'étendant longitudinalement (227) sont calibrés de manière à pouvoir coopérer et configurés pour engager et fournir une interface pouvant coulisser avec les bords de retenue se projetant vers l'intérieur (254) des rails s'étendant longitudinalement (256) des surfaces d'affichage d'indices (220, 236).

4. Seringue selon la revendication 3, dans laquelle des épaulements d'arrêt opposés et se faisant face (252) empêchent le dispositif de sécurité d'aiguille (212) d'être déplacé vers l'arrière par rapport au cylindre pour exposer à nouveau la pointe avant de l'aiguille (225) après la pointe avant de l'aiguille (225) et est recouverte par la protection de pointe d'aiguille (232) lorsque le dispositif de sécurité d'aiguille (21) se trouve dans la seconde position d'arrêt.

5. Seringue (200) selon la revendication 1, dans laquelle les indices d'échelle de dosage volumétrique sont appliqués à la surface d'affichage d'indices par tampographie.

6. Seringue (200) selon la revendication 1, dans laquelle les indices d'échelle de dosage volumétrique sont appliqués aux parois faisant face vers l'extérieur des surfaces d'affichage d'indices (220, 236) par au moins l'un d'un groupe de procédés d'application constitué du moulage, de l'estampage, du gaufrage, ou de l'impression, dans laquelle en particulier les indices d'échelle de dosage volumétrique sont appliqués à la surface d'affichage d'indices (220, 236) par tampographie.

7. Seringue (200) selon la revendication 1, dans laquelle les deux surfaces d'affichage d'indices (220, 236) parallèles, sensiblement plates, se faisant face de manière opposée, comprennent au moins un premier arrêt coulissant disposé vers l'arrière des rails s'étendant longitudinalement (256) et un second arrêt coulissant disposé vers l'avant du premier arrêt coulissant, dans laquelle en particulier une extrémité arrière du dispositif de sécurité d'aiguille (212) engage le premier arrêt coulissant dans la première position d'arrêt, et dans laquelle l'extrémité arrière du dispositif de sécurité d'aiguille (212) engage le second arrêt coulissant dans la seconde position d'arrêt pour empêcher un mouvement vers l'arrière ultérieur du dispositif de sécurité d'aiguille (212) depuis la seconde position d'arrêt vers la première position d'arrêt.

8. Seringue (200) selon la revendication 1, dans laquelle le manche d'activation (228) comprend en outre un pavé tactile (226).

9. Seringue (200) selon la revendication 1, dans laquelle la première position d'arrêt est une position d'utilisation entièrement rétractée dans laquelle la protection de pointe d'aiguille (232) entoure sensiblement le nez (234) et la pointe avant de l'aiguille (225) est exposée, dans laquelle en particulier la seconde position d'arrêt est une position post-utilisation entièrement étendue dans laquelle la protection de pointe d'aiguille (232) entoure circonférentiellement et protège la pointe avant de l'aiguille (225).

10. Seringue (200) selon la revendication 1, dans laquelle l'aiguille (225) est disposée en relation fixe par rapport au nez (234) du cylindre (210).

11. Seringue (200) selon la revendication 10, comprenant en outre : un capuchon d'aiguille amovible (214) configuré pour recouvrir la pointe avant de l'aiguille (225) lorsque le dispositif de sécurité d'aiguille (212) se trouve dans la première position d'arrêt et que le capuchon d'aiguille amovible (214) est disposé autour du nez (234) du cylindre (210).

12. Seringue (200) selon la revendication 1, dans laquelle le cylindre (210) comprend un collier faisant face vers l'arrière (230) calibré et configuré pour engager et supporter un capuchon de piston amovible (216), dans laquelle en particulier le cylindre (210) comprend une collerette de doigt se projetant transversalement (218) disposée vers l'avant du collier faisant face vers l'arrière (230).

13. Seringue (200) selon la revendication 1, dans laquelle le piston (224) comprend un joint de piston (260) qui engage la paroi latérale tubulaire de la chambre de fluide.

14. Seringue (200) selon la revendication 1, dans laquelle les indices d'échelle de dosage volumétrique chevauchent latéralement au moins une portion de la paroi latérale tubulaire de la chambre de fluide, dans laquelle en particulier les indices d'échelle volumétrique sont disposés adjacents à la portion de la paroi latérale tubulaire de la chambre de fluide de sorte que le niveau de liquide à travers la portion de la paroi latérale tubulaire et que les indices d'échelle volumétrique sont simultanément visibles.
